# EUROPEAN PATENT SPECIFICATION

(11) **EP 4 192 533 B1**
(45) Date of publication and mention of the grant of the patent: **28.08.2024**
(21) Application number: 21733568.6
(22) Date of filing: 15.06.2021
(51) Int. Cl.: A61L 15/22, A61L 15/42, A61L 15/44, A61L 15/46, B33Y 40/20, B33Y 70/00, B33Y 80/00

(54) **ADVANCED DRESSING CONTAINING A POLYCAPROLACTONE POROUS MEMBRANE**
FORTSCHRITTLICHER VERBAND MIT EINER PORÖSEN POLYCAPROLACTONMEMBRAN
PANSEMENT PERFECTIONNÉ CONTENANT UNE MEMBRANE POREUSE DE POLYCAPROLACTONE

(30) Priority: 05.08.2020 IT 202000019273
(43) Date of publication of application: 14.06.2023
(73) Proprietor: Chirlab S.r.l., 55100 Lucca (IT)
(72) Inventor: PIETRA, Daniele, I-56017 San Giuliano Terme (PI) (IT); BORGHINI, Alice, I-56017 San Giuliano Terme (PI) (IT)
(74) Representative: Long, Giorgio
(86) International application number: PCT/IB2021/055245
(87) International publication number: WO 2022/029515

(56) References cited:
- CN-A- 109 395 170
- MUWAFFAK ZAID ET AL: "Patient-specific 3D scanned and 3D printed antimicrobial polycaprolactone wound dressings", INTERNATIONAL JOURNAL OF PHARMACEUTICS, ELSEVIER, NL, vol. 527, no. 1, 29 April 2017 (2017-04-29), pages 161 - 170, XP085064659, ISSN: 0378-5173, DOI: 10.1016/J.IJPHARM.2017.04.077

## Description

### FIELD OF THE INVENTION

The present invention relates to an advanced dressing, a method for the manufacturing thereof and the use thereof, in which the advanced dressing effectively helps to promote the tissue repair process, protecting against infections and keeping the temperature and oxygen permeability of the affected tissue constant.

### BACKGROUND OF THE INVENTION

A product which promotes the tissue repair process, protects against infection and keeps the temperature and oxygen permeability of the affected tissue constant is an advanced dressing.

Advanced dressings can be divided into:
PASSIVE - Used to absorb exudates and protect the lesion from external agents.
INTERACTIVE - Interact by regulating the microenvironment of the lesion, ensuring the ideal features so that the reparative process is facilitated.
ACTIVE - Play an active role in tissue repair, sometimes modifying the cellular matrix thereof.

In the presence of skin lesions, the application of advanced dressings which promote healing can be useful. Lesions which can benefit from the application of advanced dressings are lesions of the skin and mucous membranes such as diabetic lesions and ulcers, pressure lesions and ulcers (bedsores), skin ulcers in general, surgical wounds (including at the odontostomatologic and maxillofacial level), cuts, lacerations, abrasions and bruises in general, burns.

More specifically, pressure lesions are areas of tissue damage to the skin and/or underlying tissues caused primarily by pressure, stretching, or friction. Although largely preventable, this type of damage (also called pressure ulcer, sore, ulcer or bedsore) is a significant phenomenon in hospital wards and in the territory, both for the number of patients involved and for the time and resources needed to treat the problem.

Vascular lesions of the lower limbs are defined as those skin wounds of venous, arterial and/or mixed vascular aetiology, which are localized below the knee down to the foot and which last for at least eight to ten weeks. Chronic leg ulcers are a very frequent disease in the Western world, which mainly affects the elderly, resulting in a high social cost.

Diabetic foot injuries occur when diabetic neuropathy or lower limb arterial disease compromises the function or structure of the foot. The two cases, also defined as neuropathic foot or ischaemic foot, are profoundly different from each other: however, in most subjects, especially of advanced age, both coexist giving rise to the so-called neuroischaemic foot.

The surgical wound is a tissue continuity solution produced by a mechanical agent. In clinical practice, two main types of surgical wounds can be found:
- wounds which heal at the first attempt, in which the flaps have been brought together by applying a suture. They repair quickly, generally developing a linear scar which is often not very visible;
- wounds which heal at the second attempt, in which the flaps are not joined, often due to an infection. The healing is slow and the scar which forms can vary in size.

A burn is an acute injury of traumatic origin caused by contact with a flame, with superheated liquids or solids, chemicals, electricity or radiant energy. Depending on the depth thereof, they are divided into 1st and 2nd superficial degree, 2nd deep degree and 3rd degree burns; the optimal treatment of the first two is the dressing which generally allows healing within two weeks; in the case of deep burns, the best treatment is surgery.

Various substances or products show properties useful for the regeneration of tissues affected by lesions. Furthermore, numerous attempts are known, from products on the market or from scientific and patent literature, to associate various substances in order to try to obtain effective compositions to promote the tissue repair process, protecting against infections and keeping the temperature and oxygen permeability of the affected tissue constant.

Although there are numerous known attempts (from products on the market or from scientific and patent literature) to combine various substances to try to obtain effective compositions to promote the tissue repair process, protecting against infections and keeping the temperature and oxygen permeability of the affected tissue constant, the need is still felt to be able to combine different substances with different properties, in such proportions as to exhibit synergistic effects in terms of promoting the tissue repair process, protecting from infections and keeping the temperature and oxygen permeability of the affected tissue constant and at the same time able to be well tolerated and easy to apply.

In fact, there are various technical problems which an ideal advanced dressing should solve. In particular, such compositions should:
- be effective,
- have a good bioavailability of the active ingredients,
- show very low toxicity,
- be well tolerated, comfortable and painless,
- also be adapted for prolonged and repeated uses,
- be easy to apply, for example in the form of bandages, gauze, patches, etc.,
- keep the environment in contact with the tissue lesion constantly moist,
- not be adherent to the lesion area,
- be easily removable/detachable,
- absorb unpleasant odours which can come from the lesion,
- allow the gaseous exchange of oxygen, carbon dioxide and water vapour with the environment,
- ensure the thermal insulation of the affected tissue,
- have high adsorbing capacity (exudates, microorganisms, toxic components, dead cells),
- be impermeable to the entry of external microorganisms,
- ensure mechanical protection (protect the injury from possible trauma),
- be flexible and easily adaptable to irregular anatomical contours.

Therefore, they should overall have good compliance from the subjects who are medicated therewith, for example by providing an effective dose of numerous active ingredients which act in synergy, without requiring numerous daily applications, i.e., preferably to be applied from once a week to twice a day.

### SUMMARY OF THE INVENTION

The above object has been achieved by an advanced dressing characterised by a polycaprolactone porous membrane containing:
maltodextrin,
chitosan,
collagen,
citric acid,
polyphenols and
colloidal silver.

The presence of maltodextrin favours the transfer of active ingredients to the lesion site.

The inventors of the present invention have surprisingly found that the aforesaid ingredients (maltodextrin, chitosan, collagen, citric acid, polyphenols and colloidal silver), when incorporated into a polycaprolactone porous membrane, show synergistic activity with each other in terms of healing activity (effectively helping to promote the tissue repair process), antimicrobial activity (protecting against exogenous infections) and the controlled release of active ingredients, and keeping the temperature and oxygen permeability of the affected tissue constant.

Therefore, it is an object of the present invention an advanced dressing characterised by a polycaprolactone porous membrane containing:
maltodextrin,
chitosan,
collagen,
citric acid,
polyphenols,
colloidal silver,
as outlined in the appended claims 1 to 8.

It is another object of the invention a manufacturing process of the advanced dressing membrane, as outlined in the appended claim 9.

It is another object of the invention an advanced dressing for use in effectively helping to promote the tissue repair process, protecting against exogenous infections and keeping the temperature and oxygen permeability of the affected tissue constant, as outlined in the appended claim 10.

The text of the appended claims forms an integral part of the present description.

Further features and advantages of the compositions and the process according to the invention will become apparent from the following description of embodiments thereof, given by way of indicative and non-limiting examples.

### BRIEF DESCRIPTION OF THE DRAWINGS

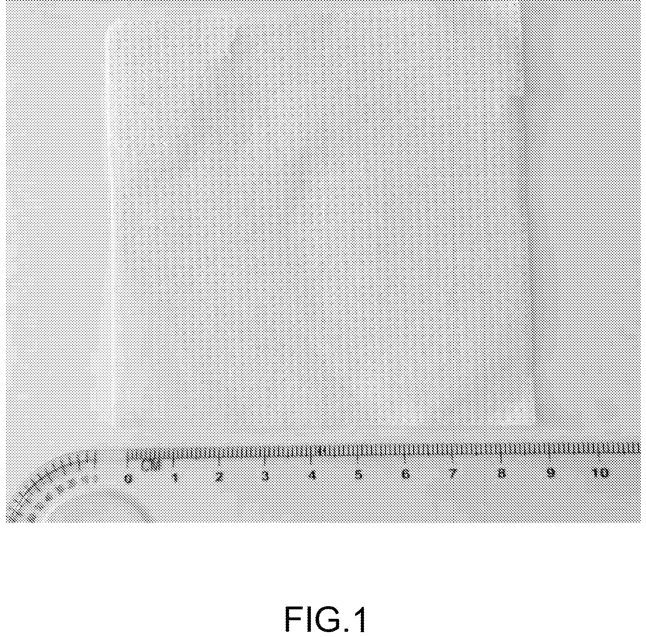

Figure 1 shows a caprolactone sheet containing maltodextrin, chitosan, collagen, citric acid, tannic acid, tea tree essential oil and colloidal silver (see Example 1).

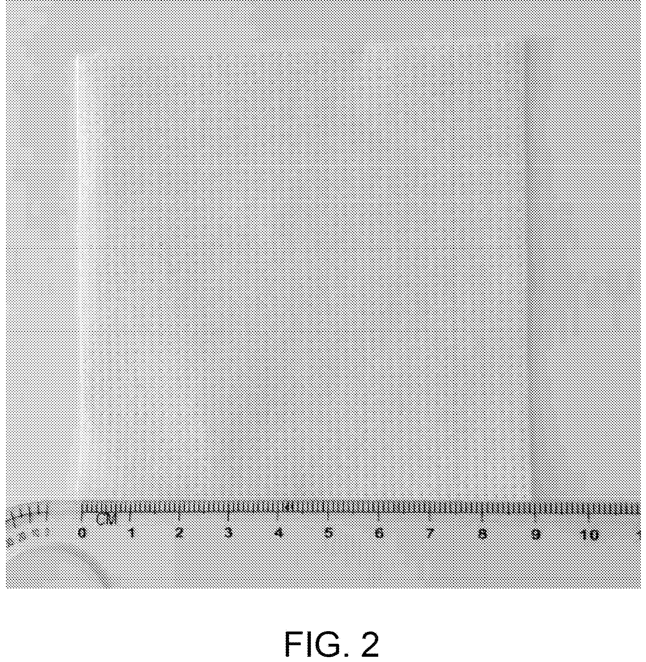

Figure 2 shows a caprolactone sheet containing chitosan, collagen, citric acid, tannic acid and colloidal silver (see reference Example 2).

### DETAILED DESCRIPTION OF THE INVENTION

According to a first embodiment, the present invention relates to an advanced dressing characterised by a polycaprolactone porous membrane containing:
maltodextrin,
chitosan,
collagen,
citric acid,
polyphenols and
colloidal silver.

According to certain embodiments of the invention, said advanced dressing comprises the following percentages by weight, calculated on the total weight of the mixture of said components:
maltodextrin from 5% to 20% by weight,
chitosan from 0,5% to 5% by weight,
collagen from 1% to 5% by weight,
citric acid from 1% to 5% by weight,
polyphenols from 0.1% to 5% by weight,
colloidal silver from 5 ppm to 50 ppm.

According to a preferred embodiment of the invention, said advanced dressing comprises the following percentages by weight, calculated on the total weight of the mixture of said components:
maltodextrin from 8% to 15% by weight,
chitosan from 1% to 3% by weight,
collagen from 2% to 4% by weight,
citric acid from 2% to 4% by weight,
polyphenols from 0.1% to 1% by weight,
colloidal silver from 5 ppm to 20 ppm.

As used herein, the term "advanced dressing" means a covering material with biocompatibility features which, by maintaining a moist microenvironment, promotes the tissue repair process, in particular an active advanced dressing.

As used herein, the term "polycaprolactone" (also referred to as PCL) means a synthetic biodegradable semi-crystalline polymer. Being provided with good biocompatibility features and high thermal stability, it is widely used in the field of biomedical applications. Polycaprolactone degrades by hydrolysis due to the presence within the structure thereof of ester bonds; since the same degradation mechanism occurs in the human body under physiological conditions, this material has received particular attention for making implantable devices. In particular, polycaprolactone is used for making long-lasting implants. The use thereof within the human body, for example as a substance releasing element, as a suture or as an adhesion element, is approved by the Food and Drug Administration.

As used herein, the term "polycaprolactone membrane" means an artificial membrane, or synthetic membrane, made of polycaprolactone as a component polymer. Such a membrane preferably has a thickness ranging from 0.01 mm to 1 mm and contains pores with a radius of 0.005 mm to 0.5 mm which allow selective exchanges of gases and substances between the tissue affected by the lesion and the external environment.

As used herein, the term "maltodextrin" means a water-soluble complex carbohydrate. It is obtained through chemical hydrolysis processes mainly from the breakdown of starches from grains (corn, oats, wheat, rice) or tubers (potatoes, tapioca). Based on the degree of transformation of these starches, maltodextrins are created, consisting of glucose molecules arranged in polymer chains of variable length. Maltodextrin is typically composed of a variety of chains which can range from 3 to 17 glucose units.

As used herein, the term "chitosan" means a linear polysaccharide composed of D-glucosamine and N-acetyl-D-glucosamine, linked via β(1-4) bonds. Chitosan is usually obtained through the deacetylation of chitin, generally extracted from the exoskeleton of crustaceans (crabs, shrimps, etc.) with aqueous solution of sodium hydroxide, or it can be of vegetable or fungal origin, i.e., obtained through the deacetylation of chitin present in fungi, in which it forms the main component of the cell wall. The features distinguishing various types of chitosans and responsible for the different properties thereof are viscosity, degree of acetylation and molecular weight. Viscosity is measured in a 1% aqueous solution of acetic acid and expressed in centipoise (cP). Viscosity is measured on a Brookfield model NDJ-1 rotational viscometer usable in a viscosity range from 10 to 100,000 cP. At room temperature, 3.0 g of test sample are taken, previously dried at constant weight at 105±2°C, before being placed in 300 ml of water with stirring. 3.0 g of glacial acetic acid are then added and stirring is continued for one hour until the sample is completely dissolved. The rotational viscometer is then used to determine the viscosity at 20±1°C. The degree of acetylation is determined using NMR spectroscopy and varies between 0% and 40% while the molecular weight range varies between 3,800 and 20,000 Dalton.

As used herein, the term "collagen" means the body's most abundant protein, where it is concentrated primarily in the bones, tendons, cartilage, skin, membranes and blood vessels. In particular, collagen is one of the main components of connective tissue. At the cutaneous level, collagen contributes to maintaining skin firmness, tone and turgidity. It is present in the dermis where, together with elastic fibres and glycosaminoglycans, it gives rise to that three-dimensional structure which supports and sustains the skin, giving it strength and elasticity.

As used herein, the term "citric acid" means 2-hydroxy-1,2,3-propanetricarboxylic acid. It appears as a solid, colourless substance with a raw formula C₆H₈O₇, soluble in water over a wide pH range. It can be found in fruit, especially of the genus *Citrus.*

As used herein, the term "polyphenols" means the total amount of compounds with a polyphenolic structure, expressed as caffeic acid equivalents, and determined analytically by the Folin-Ciocalteu method [Ainsworth EA and Gillespie KM. Estimation of total phenolic content and other oxidation substrates in plant tissues using Folin-Ciocalteu reagent. Nature Protocols 2007;2:875-875]. Non-limiting examples of polyphenols are tannic acid, quercetin, apigenin, resveratrol, isoflavones, catechins. Non-limiting examples of polyphenol sources are plant extracts such as green tea extract, acai fruit extract, red vine extract, pomegranate extract, olive leaf extract, etc.

As used herein, the term "colloidal silver" means a compound based on silver particles. It is presented as flakes or granular powder which are bright grey-black-blue in colour, substantially odourless, insoluble in alcohol and ether, slightly soluble in water.

As used herein, the term "vegetable oil" means an oil obtained from a vegetable source. In the present invention a vegetable oil can be a fixed oil (obtained by mechanical pressing or extraction with apolar solvents and subsequent evaporation) or an essential oil (obtained by steam distillation or hydrodistillation, or extraction in supercritical fluids) and can be, for example, in the form of oil as such, of microencapsulated or carrier-adsorbed oil (for example cyclodextrin) or of powdered oil.

The components of the present formulation are all known and individually available on the market.

The advanced dressing of the invention can also comprise further ingredients and excipients such as, but not limited to, vegetable oils, anaesthetic active ingredients, analgesic and anti-inflammatory active ingredients, antimicrobial active ingredients, growth factors, cytokines, hormones, vitamins, minerals, plant extracts, emollients, humectants, binders, rheology modifiers, pH regulators, preservatives, flavourings, water and the like. For example, the advanced dressing of the invention can comprise pharmaceutically acceptable excipients and carriers such as potassium sorbate, sodium benzoate, sucralose, maltodextrin, microcrystalline cellulose, Arabic gum, magnesium stearate, silica, sorbitol and the like.

Active anaesthetic ingredients are compounds or substances of synthetic or natural origin with local anaesthetic activity. Non-limiting examples of synthetic anaesthetic active ingredients are procaine, chlorprocaine, lidocaine, prilocaine, bupivacaine, ropivacaine, levobupivacaine and the like. Non-limiting examples of anaesthetic active ingredients of natural origin are mint essential oil, myrrh essential oil, eucalyptus essential oil, and the like.

Analgesic and anti-inflammatory active ingredients are compounds or substances of synthetic or natural origin with analgesic and anti-inflammatory activity. Non-limiting examples of synthetic analgesic and anti-inflammatory active ingredients are steroidal (for example cortisone drugs and the like) and non-steroidal (for example NSAIDs and the like) . Non-limiting examples of analgesic and anti-inflammatory active ingredients of natural origin are those contained in birch extracts (*Betula pendula*), cloves (*Syzygium aromaticum*), spirea (*Spirea ulmaria*), willow (*Salix alba*), and the like.

Non-limiting examples of antimicrobial active ingredients are bacteriostatic, bactericidal, fungistatic, fungicidal, virustatic, virucidal, and similar drugs, of both synthetic and natural origin.

Non-limiting examples of vegetable oils are essential oils with antimicrobial activity, such as tea tree oil (*Melaleuca* spp.), or algal oils rich in polyunsaturated fatty acids (PUFA), or oils with anti-inflammatory activity, such as copaiba oleoresin (*Copaifera* spp).

The advanced dressing can also contain additional excipients or other active ingredients depending on the type of formulation to be prepared.

In certain embodiments, the advanced dressing is in the form of a patch.

In certain embodiments, the advanced dressing is in sheet or film form.

There are no particular restrictions on the shape and size of the advanced dressing of the invention, which can be, for example, square, rectangular, round, elliptical, etc. and varying in size from a few millimetres to about one metre.

### EXAMPLES

### Example 1 - Advanced dressing in sheet form with maltodextrin

An advanced sheet dressing was prepared using the following ingredients (Table 1):

**Table 1**

| **RAW MATERIAL** | **Quantity** |
|---|---|
| Maltodextrin | 500 g |
| Collagen | 150 g |
| Citric acid | 150 g |
| Chitosan | 100 g |
| Tannic acid | 20 g |
| Melaleuca essential oil | 1.5 g |
| Colloidal silver 50 mg | 50 mg |
| Polycaprolactone | as needed to 5000 g |

The manufacturing process of the aforesaid advanced dressing is summarised below:
- finding in the international market the PCL with a particle size compatible with the additive manufacturing process by means of the laser sintering technique starting from a powder,
- CAD design of the parts to be made and export of files in IGES - STEP format to allow the subsequent processing thereof with software responsible for managing the "machine space",
- preparation of the mixture containing PCL and the other active ingredients and excipients,
- sintering process with the generation of parts immersed in a bed of unsintered powder,
- separation of the details made by the polycaprolactone powder,
- cleaning of the pieces by blowing, use of a vibrating surface, ultrasonic cleaning in alcoholic solution at a controlled temperature,
- control of details, sterilization and packaging.

### Example 2 (Reference example) - advanced dressing in sheet form without maltodextrin

An advanced dressing was prepared using the following ingredients (Table 2):

**Table 2.**

| **RAW MATERIAL** | **Quantity** |
|---|---|
| Collagen | 150 g |
| Citric acid | 150 g |
| Chitosan | 100 g |
| Tannic acid | 20 g |
| Colloidal silver | 50 mg |
| Polycaprolactone | as needed to 5000 g |

The manufacturing process of the aforesaid advanced dressing is summarised below:
- finding in the international market the PCL with a particle size compatible with the additive manufacturing process by means of the laser sintering technique starting from a powder,
- CAD design of the parts to be made and export of files in IGES - STEP format to allow the subsequent processing thereof with software responsible for managing the "machine space",
- preparation of the mixture containing PCL and the other active ingredients and excipients,
- sintering process with the generation of parts immersed in a bed of unsintered powder,
- separation of the details made by the polycaprolactone powder,
- cleaning of the pieces by blowing, use of a vibrating surface, ultrasonic cleaning in alcoholic solution at a controlled temperature,
- control of details, sterilization and packaging.

### Example 3 - Release of the active ingredients from an advanced dressing containing a polycaprolactone porous membrane

A square-shaped sample of 9 cm per side was taken from a polycaprolactone membrane prepared as in Example 1. An analogous sample was taken from a polycaprolactone membrane prepared as in reference Example 2. Each sample was separately placed in a Petri dish, immersed in 40 ml of demineralized water to evaluate how much active ingredient (polyphenols) was released over time. After 48 hours, the water contained in each Petri dish was subjected to the total polyphenol dosage (performed by the Folin-Ciocalteau assay as described above) which gave the results reported in Table 3:

**Table 3**

| **Example** | **Total polyphenol concentration*** |
|---|---|
| 1 | 23.2 mg/kg |
| 2 (Reference) | 15.7 mg/kg |

| | |
|---|---|
| * Total polyphenols expressed as caffeic acid equivalents. | |

Table 3 shows that the release of active ingredient from the advanced dressing containing a polycaprolactone membrane is more effective in Example 1 with respect to reference Example 2. This confirms that the presence of maltodextrin facilitates the release of active ingredients from such an advanced dressing.

## Claims

1. An advanced dressing **characterised by** a polycaprolactone porous membrane comprising:
maltodextrin
chitosan
collagene
citric acid
polyphenols
colloidal silver.

2. The advanced dressing according to claim 1, wherein the polycaprolactone porous membrane contains:
maltodextrin from 5% to 20% by weight,
chitosan from 0,5% to 5% by weight,
collagene from 1% to 5% by weight,
citric acid from 1% to 5% by weight,
polyphenols from 0,1% to 5% by weight,
colloidal silver from 5 ppm to 50 ppm.

3. The advanced dressing according to claim 1 or 2, wherein the polycaprolactone porous membrane contains:
maltodextrin from 8% to 15% by weight,
chitosan from 1% to 3% by weight,
collagene from 2% to 4% by weight,
citric acid from 2% to 4% by weight,
polyphenols from 0,1% to 1% by weight,
colloidal silver from 5 ppm to 20 ppm.

4. The advanced dressing according to anyone of claims 1-3 wherein polyphenols are tannic acid.

5. The advanced dressing according to anyone of claims 1-4 further comprising a vegetable oil selected from the group consisting of *Melaleuca* spp. (tea tree) essential oil, *Syzygium aromaticum* (clove) essential oil, mint essential oil, myrrh essential oil, eucalyptus essential oil, *Copaifera* spp. oleoresin, wherein the vegetable oil is from 0,01% to 5% by weight.

6. The advanced dressing according to anyone of claims 1-5, wherein the polycaprolactone porous membrane is from 0,01 mm to 1 mm thick and contains pores with a radius from 0,005 mm to 0,5 mm.

7. The advanced dressing according to anyone of claims 1-6, wherein the advanced dressing further comprises additional layers made up of exudates absorbing materials.

8. The advanced dressing according to anyone of claims 1-6, wherein the advanced dressing is in the form of a patch.

9. A process for the manufacturing of the dressing according to anyone of claims 1-8, said process comprising the following steps:
- finding in the international market the PCL with a particle size compatible with the additive manufacturing process by means of the laser sintering technique starting from a powder,
- CAD design of the parts to be made and export of files in IGES - STEP format to allow their subsequent processing with software that is responsible for managing the "machine space",
- preparation of the mixture containing PCL and the other active ingredients and excipients,
- sintering process with the generation of parts immersed in an bed of unsintered powder,
- separation of the details made by the powder of polycaprolactone,
- cleaning of the pieces by blowing, use of a vibrating surface, ultrasonic cleaning in alcoholic solution at a controlled temperature,
- control of details, sterilization and packaging.

10. The advanced dressing according to anyone of claims 1-8, for use in promoting the tissue repair process.

## Patentansprüche

1. Fortschrittlicher Verband, **gekennzeichnet durch** eine poröse Polycaprolacton-Membran, umfassend:
Maltodextrin
Chitosan
Kollagene
Zitronensäure
Polyphenole
kolloidales Silber.

2. Fortschrittlicher Verband nach Anspruch 1, wobei die poröse Polycaprolacton-Membran enthält:
Maltodextrin von 5 bis 20 Gew.-%,
Chitosan von 0,5 bis 5 Gew.-%,
Kollagene von 1 bis 5 Gew.-%,
Zitronensäure von 1 bis 5 Gew.-%,
Polyphenole von 0,1 bis 5 Gew.-%,
kolloidales Silber von 5 ppm bis 50 ppm.

3. Fortschrittlicher Verband nach Anspruch 1 oder 2, wobei die poröse Polycaprolacton-Membran enthält:
Maltodextrin von 8 bis 15 Gew.-%,
Chitosan von 1 bis 3 Gew.-%,
Kollagene von 2 bis 4 Gew.-%,
Zitronensäure von 2 bis 4 Gew.-%,
Polyphenole von 0,1 bis 1 Gew.-%,
kolloidales Silber von 5 ppm bis 20 ppm.

4. Fortschrittlicher Verband nach einem der Ansprüche 1-3, wobei die Polyphenole Gerbsäure sind.

5. Fortschrittlicher Verband nach einem der Ansprüche 1-4, ferner umfassend ein Pflanzenöl, ausgewählt aus der Gruppe bestehend aus ätherischem Öl von *Melaleuca* spp. (Teebaum), ätherischem Öl von *Syzygium aromaticum* (Gewürznelke), ätherischem Minzöl, ätherischem Myrrheöl, ätherischem Eukalyptusöl, *Copaifera spp.* Oleoresin, wobei das Pflanzenöl 0,01 bis 5 Gew.-% ausmacht.

6. Fortschrittlicher Verband nach einem der Ansprüche 1-5, wobei die poröse Polycaprolacton-Membran 0,01 mm bis 1 mm dick ist und Poren mit einem Radius von 0,005 mm bis 0,5 mm enthält.

7. Fortschrittlicher Verband nach einem der Ansprüche 1-6, wobei der fortschrittliche Verband außerdem zusätzliche Schichten aus Exsudat-absorbierenden Materialien umfasst.

8. Fortschrittlicher Verband nach einem der Ansprüche 1-6, wobei der fortschrittliche Verband in Form eines Pflasters vorliegt.

9. Verfahren zur Herstellung des Verbandes nach einem der Ansprüche 1-8, wobei das Verfahren die folgenden Schritte umfasst:
- Auffinden des PCL mit einer Teilchengröße, die mit dem additiven Herstellungsverfahren mittels der Lasersintertechnik, ausgehend von einem Pulver, kompatibel ist, auf dem internationalen Markt,
- CAD-Design der herzustellenden Teile und Export der Dateien im ICES STEP-Format, um ihre spätere Verarbeitung mit einer Software zu ermöglichen, die für die Verwaltung des "Maschinenraums" zuständig ist,
- Herstellung der Mischung, die PCL und die anderen Wirkbestandteile und Hilfsstoffe enthält,
- Sinterprozess mit der Erzeugung von Teilen, die in ein Bett aus ungesintertem Pulver getaucht sind,
- Trennung der aus dem Polycaprolacton-Pulver hergestellten Teile,
- Reinigung der Teile durch Ausblasen, Verwendung einer vibrierenden Oberfläche, Reinigung mit Ultraschall in alkoholischer Lösung bei kontrollierter Temperatur,
- Kontrolle der Details, Sterilisation und Verpackung.

10. Fortschrittlicher Verband nach einem der Ansprüche 1-8 zur Verwendung bei der Förderung des Gewebereparaturprozesses.

## Revendications

1. Pansement perfectionné **caractérisé par** une membrane poreuse de polycaprolactone comprenant :
maltodextrine
chitosane
collagène
acide citrique
polyphénols
argent colloïdal.

2. Pansement perfectionné selon la revendication 1, dans lequel la membrane poreuse de polycaprolactone contient :
maltodextrine de 5 % à 20 % en poids,
chitosane de 0,5 % à 5 % en poids,
collagène de 1 % à 5 % en poids,
acide citrique de 1 à 5 % en poids,
polyphénols de 0,1 % à 5 % en poids,
argent colloïdal de 5 ppm à 50 ppm.

3. Pansement perfectionné selon la revendication 1 ou 2, dans lequel la membrane poreuse de polycaprolactone contient :
maltodextrine de 8 % à 15 % en poids,
chitosane de 1 % à 3 % en poids,
collagène de 2 % à 4 % en poids,
acide citrique de 2 % à 4 % en poids,
polyphénols de 0,1 % à 1 % en poids,
argent colloïdal de 5 ppm à 20 ppm.

4. Pansement perfectionné selon l'une quelconque des revendications 1 à 3, dans lequel les polyphénols sont de l'acide tannique.

5. Pansement perfectionné selon l'une quelconque des revendications 1 à 4, comprenant en outre une huile végétale sélectionnée dans le groupe constitué d'huile essentielle de *Melaleuca* spp. (arbre à thé), huile essentielle de *Syzygium aromaticum* (clou de girofle), huile essentielle de menthe, huile essentielle de myrrhe, huile essentielle d'eucalyptus, oléorésine de *Copaifera* spp., dans lequel l'huile végétale est de 0,01 % à 5 % en poids.

6. Pansement perfectionné selon l'une quelconque des revendications 1 à 5, dans lequel la membrane poreuse de polycaprolactone a une épaisseur de 0,01 mm à 1 mm et contient des pores ayant un rayon de 0,005 mm à 0,5 mm.

7. Pansement perfectionné selon l'une quelconque des revendications 1 à 6, dans lequel le pansement perfectionné comprend en outre des couches supplémentaires constituées de matériaux absorbant des exsudats.

8. Pansement perfectionné selon l'une quelconque des revendications 1 à 6, dans lequel le pansement perfectionné est sous la forme d'un patch.

9. Procédé pour la fabrication du pansement selon l'une quelconque des revendications 1 à 8, ledit procédé comprenant les étapes suivantes :
- recherche, sur le marché international, du PCL ayant une taille de particule compatible avec le procédé de fabrication additive au moyen de la technique de frittage laser en partant d'une poudre,
- conception CAO des parties à réaliser et export des fichiers au format IGES - STEP pour permettre leur traitement ultérieur avec un logiciel qui est chargé de gérer « l'espace machine »,
- préparation du mélange contenant le PCL et les autres principes actifs et excipients,
- procédé de frittage avec la génération de parties immergées dans un lit de poudre non frittée,
- séparation des détails réalisés par la poudre de polycaprolactone,
- nettoyage des pièces par soufflage, utilisation d'une surface vibrante, nettoyage aux ultrasons en solution alcoolique à température régulée,
- régulation des détails, stérilisation et conditionnement.

10. Pansement perfectionné selon l'une quelconque des revendications 1 à 8, pour son utilisation afin de favoriser le processus de réparation des tissus.
